# EUROPEAN PATENT APPLICATION

(11) **EP 1 820 791 A1**
(43) Date of publication of application: **22.08.2007**
(21) Application number: 07000901.4
(22) Date of filing: 17.01.2007
(51) Int. Cl.: C07C 51/50, C07C 57/04

(54) **Method of manufacturing (meth)acrylic acid**

(30) Priority: 19.01.2006 JP 2006011594
(71) Applicant: NIPPON SHOKUBAI CO., LTD., Osaka-shi, Osaka (JP)
(72) Inventor: Ishikawa, Masaru, Aboshi-ku Himeji-shi, Hyogo (JP); Ishii, Yoshitake, Aboshi-ku Himeji-shi, Hyogo (JP)
(74) Representative: Müller-Boré & Partner Patentanwälte

(57) **Abstract**

A process for producing (meth)acrylic acid while preventing a polymerization of (meth)acrylic acid using a polymerization inhibitor solution, comprising the steps of obtaining a (meth)acrylic solution containing formaldehyde from any step of the process for producing (meth)acrylic acid to use as a solvent of the polymerization inhibitor solution, and adding phenothiazine or derivatives thereof and/or hydroquinone or derivatives thereof to the (meth)acrylic solution containing formaldehyde to obtain polymerization inhibitor solution so that the polymerization inhibitor solution contains 0.1 mass % to 3 mass % of phenothiazine or derivatives thereof and/or 0.1 mass % to 10 mass % of hydroquinone or derivatives thereof, wherein the polymerization inhibitor solution contains 10 ppm to 5000 ppm (based on mass) of formaldehyde. The polymerization inhibitor solution used in the present invention is stable, generates little precipitate and is prepared using a low purity (meth)acrylic acid solution from the process for producing (meth)acrylic acid.

## Description

The present invention relates to a process for producing (meth)acrylic acid while preventing a polymerization using a polymerization inhibitor solution, and a solvent of the polymerization inhibitor solution is prepared at any step in the process for producing (meth)acrylic acid containing a step of generating formaldehyde as a by-product. The polymerization inhibitor solution used in the invention is stabilized and usable at any process.

It is general that acrylic acid is industrially produced by a propylene oxidation process in which propylene and/or acrolein is oxidized by a gas-phase catalytic oxidation. When acrylic acid is produced by the propylene oxidation process, impurities such as water; acids such as propionic acid, acetic acid and maleic acid; aldehydes such as acrolein, furfural and formaldehyde; ketones such as acetone and diketones are generated as by-products. (Meth)acrylic acid can be synthesized by a gas-phase catalytic oxidation of raw materials such as isobutylene and/or t-butyl alcohol. In this case, impurities such as water; maleic acid; aromatic carboxylic acids such as terephthalic acid; aldehydes such as formaldehyde and (meth)acrolein; ketones such as acetone and diketones are generated as by-products. The gas containing these by-products is colleted as an acrylic acid-containing solution by contacting with a collecting solution, and the collected solution is purified and separated by a process such as distillation.

Since (meth)acrylic acid is easily polymerized by heating, various polymerization inhibitors are generally added at a distillation process to inhibit a polymerization of (meth)acrylic acid for the purpose of continuous stable operation of an apparatus.

Various compounds are used as the polymerization inhibitor, and phenothiazine or derivatives thereof are also known to be effective for preventing the polymerization of (meth)acrylic acid. Since phenothiazine or derivatives thereof are solid at normal temperature, they are often used as a solution which is dissolved by some solvent. For example, JP-A-H8-40974 describes that the polymerization inhibitor solution which is dissolved by acrylic acid, an azeotropic agent and water is fed to a top portion of a distillation column when azeotropic distillation is carried out. JP-A-2003-113138 describes an invention that an inner wall surface of a distillation column is preliminarily wetted with the polymerization inhibitor solution, and a mixture of water and toluene, a mixture of water and acrylic acid, and crude acrylic acid (a bottom liquid of an acrylic acid distillation column) containing dimer and trimer of acrylic acid can be used as a solvent of the polymerization inhibitor solution in addition to water and an organic solvent such as alcohol and toluene.

In the case that only water or an organic solvent such as alcohol and toluene shown in the above-mentioned conventional art is used as a solvent, adding a compound other than an objective product of (meth)acrylic acid in the process for producing (meth)acrylic acid is not efficient considering a purification step. Producing of the polymerization inhibitor solution using high purity (meth)acrylic acid is also not efficient because an objective product of (meth)acrylic acid is added in the process for producing (meth)acrylic acid. Consequently, it is considered to be most efficient that phenothiazine or derivatives thereof solution are prepared using a comparatively low purity (meth)acrylic acid solution (bottom liquid of the column, condensate and the like) that is produced at any step of the process for producing (meth)acrylic acid. Therefore, the present inventors have carried out experiments, and generation of a precipitate was confirmed. When the precipitate is left, troubles such as generation of a scale in a distillation column and blockage of pipe occur, and a continuous stable operation is interfered. However, even if the precipitate was removed from the polymerization inhibitor solution by a strainer and the like, the effect of the polymerization inhibition concerning to the concentration of phenothiazine dissolved at the beginning was not obtained. This suggested that phenothiazine was converted to the precipitate.

Furthermore, it was confirmed that hydroquinone or derivatives thereof represented by hydroquinone and methoquinone (hydroquinone mono methyl ether) formed a precipitate depending on conditions of temperature and its concentration.

Based on the above-mentioned knowledge, subjects in the present invention are set as follows: (1) finding out a cause of the generation of the precipitate; (2) finding a method for suppressing the generation of the precipitate; (3) providing a process for producing (meth)acrylic acid while preventing the polymerization using the polymerization inhibitor solution which is stable, generates little precipitate and is prepared using a low purity (meth)acrylic acid solution from the (meth)acrylic acid producing process.

The present inventors have ascertained that the above-mentioned precipitate was generated when formaldehyde was contained in the (meth)acrylic acid solution produced in the producing process and have completed the present invention.

The present invention is a process for producing (meth)acrylic acid while preventing the polymerization of (meth)acrylic acid using polymerization inhibitor solution, comprising the steps of: obtaining a (meth)acrylic solution containing formaldehyde from any step of the process for producing (meth)acrylic acid to use as a solvent of the polymerization inhibitor solution, and adding phenothiazine or derivatives thereof and/or hydroquinone or derivatives thereof to the (meth)acrylic solution containing formaldehyde to obtain polymerization inhibitor solution so that the polymerization inhibitor solution contains 0.1 mass % to 3 mass % of phenothiazine or derivatives thereof and/or 0.1 mass % to 10 mass % of hydroquinone or derivatives thereof, wherein the polymerization inhibitor solution contains 10 ppm to 5000 ppm (based on mass) of formaldehyde.

The concentration of the formaldehyde is preferably 10 ppm to 2000 ppm (based on mass) in the polymerization inhibitor solution.

Preparation and preservation of the polymerization inhibitor solution is preferably carried out at 50°C or less. A polymerization inhibitor other than phenothiazine or derivatives thereof and hydroquinone or derivatives thereof may be added when obtaining the polymerization inhibitor solution. The (meth)acrylic acid solution containing formaldehyde is preferably a solution containing 1 mass % to 99.9 mass % of (meth)acrylic acid.

The polymerization inhibitor solution is preferably fed to a column apparatus used in the process for producing (meth)acrylic acid. In particular, it is recommended that the polymerization inhibitor solution is preferably fed to one or more of a collection column, a condenser and a distillation column that are the column apparatuses operated at high temperature.

In the present invention, the "process for producing (meth)acrylic acid" means a process for producing acrylic acid or methacrylic acid.

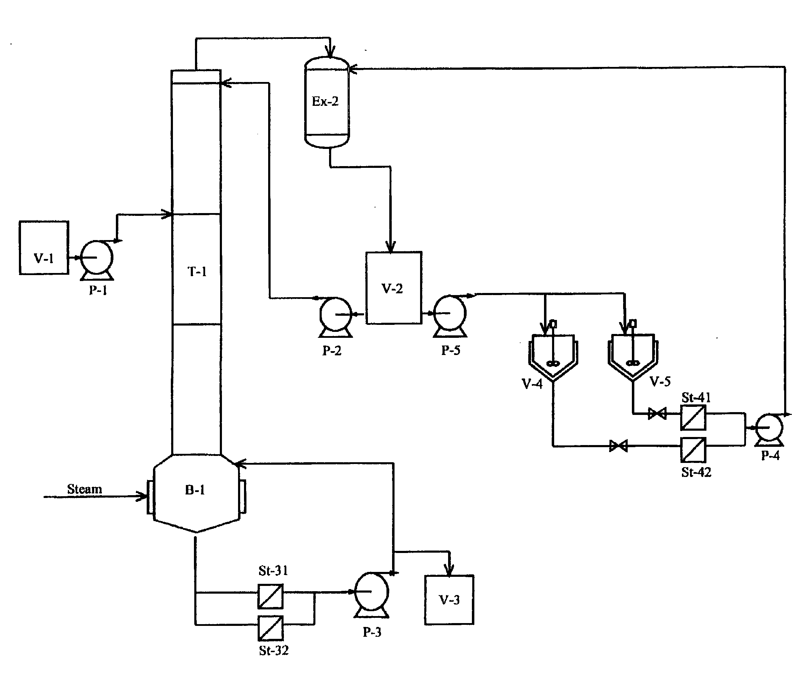

Fig. 1 is a flow sheet of a distillation facilities used in Experiment 5 to Experiment 7.

As described above, when (meth)acrylic acid is prepared by a propylene oxidation process, formaldehyde is inevitably generated as a by-product. The present inventors have examined a generation factor of the precipitate in phenothiazine or derivatives thereof solution. The precipitate is generated in a short time of 1 hour to 5 hours when phenothiazine is added exceeding 3 mass % (the concentration in the polymerization inhibitor solution, and hereinafter the same) and the concentration of formaldehyde is more than 5000 ppm (the concentration in the polymerization inhibitor solution; based by mass, and hereinafter the same). The precipitate is white or gray fine particles and floated when the solution is slightly stirred, but it precipitates at the bottom of a vessel when the solution is left. The amount of the precipitate is increased in accordance with the increase of the concentration of formaldehyde when the solubilized amount of phenothiazine is the same. The precipitate is not dissolved again even if the solution is stirred at about 50°C for one day and night or more. The precipitate is not preferable because it causes a blockage in a producing equipment.

Thus, it is preferable that the concentration of phenothiazine or derivatives thereof is 3 mass % or less and the concentration of formaldehyde is 5000 ppm or less when preparing the polymerization inhibitor solution. The lower limit of the concentration of phenothiazine or derivatives thereof is not specifically defined, but when it is too low, a large vessel for dissolution is required and the amount of the polymerization inhibitor solution becomes large; therefore, it is preferable that the concentration of phenothiazine or derivatives thereof is 0.1 mass % or more. Furthermore, it is more preferable that the upper limit of the concentration of phenothiazine or derivatives thereof is 2 mass %.

Since it is preferable that the concentration of formaldehyde is as little as possible, a preferable upper limit of the concentration of formaldehyde is 2000 ppm and a more preferable upper limit is 500 ppm. However, when the concentration of formaldehyde is less than 10 ppm, the precipitate is not generated even if phenothiazine is added by exceeding 3 mass %. Therefore, the lower limit of the concentration of formaldehyde is set to 10 ppm in order to clarify the effect of the invention.

The derivatives of phenothiazine include phenothiazine having a substituent. The substituent includes alkyl group such as a methyl group and an ethyl group; alkoxy group such as a methoxy group and an ethoxy group; halogen atom such as bromine atom and a chlorine atom and the like. Phenothiazine having one or two of these substituents can be used. Only one kind of phenothiazine or derivatives thereof may be used, and the mixture of two or more kinds of them may be used. Phenothiazine or derivatives thereof that are available from Avecia Limited and have a prill shape (tiny sphere) are preferably used because they are easily transferred from a feeder or a hopper and a blocking is hardly happened.

Hydroquinone or derivatives thereof such as hydroquinone, methoquinone also generate a precipitate like in the case of phenothiazine or derivatives thereof when formaldehyde is concluded in the (meth)acrylic acid solution. Hydroquinone or derivatives thereof are easily precipitated when the concentration of hydroquinone or derivatives thereof exceeds 10 mass % in the polymerization inhibitor solution. Thus, the concentration of hydroquinone or derivatives thereof is preferably 0.1 mass % to 10 mass % in the polymerization inhibitor solution. The concentration of formaldehyde is preferably adjusted to 10 ppm to 5000 ppm in the polymerization inhibitor solution because the amount of precipitate tends to increase in accordance with the concentration of formaldehyde in the polymerization inhibitor solution like in the case of phenothiazine. In the case of using hydroquinone or derivatives thereof, it is preferable that the polymerization inhibitor solution is prepared and preserved at 50°C or less because the precipitation is suppressed irrespective of the concentration of formaldehyde. When the temperature at preparation and/or preservation of the polymerization inhibitor solution exceeds 50°C, hydroquinone or derivatives thereof easily causes generation of the precipitate. This tendency becomes remarkable when the concentration of formaldehyde and hydroquinone or derivatives thereof is higher.

A polymerization inhibitor other than phenothiazine or derivatives thereof and/or hydroquinone or derivatives thereof may be contained in the polymerization inhibitor solution, in addition to phenothiazine or derivatives thereof and/or hydroquinone or derivatives thereof. For example, N-oxyl compounds such as 4-hydroxy-2, 2, 6, 6-tetramethylpiperidinooxyl; manganese salts such as manganese acetate; copper salts of dialkyl (or diphenyl) dithiocarbamate (an alkyl group; methyl, ethyl, propyl, butyl and the like) such as copper dibutylthiocarbamate; nitroso compounds; amine compounds such as p-phenylenediamine; tetraalkyl (or phenyl) thiuramdisulfide (an alkyl group; methyl, ethyl, propyl, butyl and the like); methylene blue and the like are indicated. Since the polymerization inhibitors indicated above are not concerned with generation of the precipitate, they may be dissolved at an amount of exhibiting effect as a polymerization inhibitor.

In the present invention, a solvent of the polymerization inhibitor solution is a (meth)acrylic acid solution prepared at any step of the process for producing (meth)acrylic acid. Herein, the process for producing (meth)acrylic acid comprises the following steps (1) to (3) after a gas-phase catalytic oxidation process.
(1) A step of obtaining the (meth)acrylic acid solution from (meth)acrylic acid-containing mixed gas: the step includes, for example, (a) a step of condensing the (meth)acrylic acid-containing mixed gas by cooling to obtain the (meth)acrylic acid solution, (b) a step of contacting the (meth)acrylic acid-containing mixed gas with water or a high boiling point solvent (diphenyl ether, diphenyl and the like) to obtain the (meth)acrylic acid solution, (c) a step of collecting the (meth)acrylic acid-containing gas using the (meth)acrylic acid solution obtained in the step (1) or in other steps to obtain the (meth)acrylic acid solution with higher concentration, and the like. Furthermore, a step of using a recycle gas extracted from a top of a column for a reaction and condensing a part of the gas, and a stripping step of removing unreacted (meth)acrolein from the (meth)acrylic acid solution which is condensed or collected are also included.
(2) A step of distilling the (meth)acrylic acid solution in the presence of an azeotropic solvent to obtain crude (meth)acrylic acid.
(3) A step of refining the crude (meth)acrylic acid: a step of refining the crude (meth)acrylic acid by distillation, stripping, crystallization, extraction, absorption, partial condensation and the like, or a combination of them. Distillation includes all distillation steps used generally in a producing high purity (meth)acrylic acid such as a high boiling point component separation step for separating high boiling point components, a low boiling point component separation step for separating low boiling point components such as acetic acid, an aldehyde or derivatives thereof removal step for removing unreacted (meth)acrolein or other aldehyde derivatives, and a maleic acid separation step for separating a by-product of maleic acid.

Various modification of above-mentioned methods used generally in the producing of high purity (meth)acrylic acid are also included in the steps (1) to (3).

In the above-mentioned step (1) of obtaining the (meth)acrylic acid solution, a bottom liquid of a column, a collecting solution, a solution extracted from a middle portion of a column (a side cut solution), a reflux solution, a condensate obtained by cooling a gas discharged from a top portion of a column and the like can be used as the solvent for the polymerization inhibitor solution. When a stripping step for removing (meth)acrolein is adopted, a bottom liquid of a column, a solution extracted from a middle portion of a column and the like can be also utilized.

In the above-mentioned azeotropic distillation step (2), a feed solution, a bottom liquid of a column, a solution extracted from a middle portion of a column, a reflux solution, a distillate and the like can be used as the solvent for the polymerization inhibitor solution.

In the above-mentioned purification step (3), a bottom liquid of a column, a solution extracted from a middle portion of a column, a reflux solution, a distillate and the like in a distillation step and a stripping step; a residual mother solution, a purification solution, a partially melt solution after crystallization and the like in a crystallization step; a feed solution, an extract solution, an extract residue solution and the like in an extraction step; a bottom liquid of a column, a collecting solution, a solution extracted from a middle portion of a column, a condensate obtained by cooling a gas discharged from a top portion of a column and the like in an absorption step; and a condensate and the like in a partial condensation step can be respectively utilized as the solvent for the polymerization inhibitor solution.

For example, when a waste oil (Michael adduct and the like are contained) decomposition step is carried out after the step (1), a distillate, a reflux solution and the like in the waste oil decomposition step can be used as the solvent for the polymerization inhibitor solution. When the decomposition solution of the waste oil is used as the solvent, there is a merit that a solubility of phenothiazine in the solvent is increased because of little containing of water and the influence of impurities in the waste oil. However, the solvent is not limited to this.

One or more kind of the (meth)acrylic acid solution prepared at any step of the process for producing (meth)acrylic acid can be used as the solvent for the above-mentioned polymerization inhibitor solution. The polymerization inhibitor solution obtained can be also used to inhibit the polymerization of (meth)acrylic acid in any step, as will be described later.

The above-mentioned various solvents for the polymerization inhibitor solution preferably include 1 mass % to 99.9 mass % of (meth)acrylic acid. When the concentration of (meth)acrylic acid is too low, it is inefficient because the mass of unnecessary substances is large in the polymerization inhibitor solution. Components other than (meth)acrylic acid in the (meth)acrylic acid solution prepared in the step (1) are shown as followings: water which is contained in the case of condensing a reaction mixed gas by cooling or in the case of using water as a collecting solution; and diphenyl ether, diphenyl and the like which are contained in the case of using a high boiling point solvent as a collecting solution, in addition to various by-products. In the case that the azeotropic step is adopted, azeotropic solvents such as toluene and methyl isobutyl ketone are occasionally contained.

When only phenothiazine or derivatives thereof or both of phenothiazine or derivatives thereof and hydroquinone or derivatives thereof are used as the polymerization inhibitor, it is preferable that the (meth)acrylic acid containing 50 mass % to 99.9 mass % of (meth)acrylic acid are used as the solvent. A more preferable lower limit of the concentration of (meth)acrylic acid is 70 mass %. Furthermore, since the less water content is, the more easily phenothiazine or derivatives thereof are dissolved, it is preferable that the amount of water is 0.01 mass % to 30 mass % (a more preferable upper limit is 20 mass %).

When only hydroquinone or derivatives thereof are used as the polymerization inhibitor, it is preferable that the (meth)acrylic acid containing 1 mass % to 50 mass % of (meth)acrylic acid are used as the solvent. Since the more water is concluded in the (meth)acrylic acid solution, the more a solubility of hydroquinone or derivatives thereof is increased, it is preferable that rest of (meth)acrylic acid is water. A more preferable upper limit of the concentration of (meth)acrylic acid is 20 mass %.

In order to prepare the polymerization inhibitor solution, one or more kind of (meth)acrylic acid solutions is extracted at any step in the producing process to be fed to a dissolution vessel, and phenothiazine or derivatives thereof and/or hydroquinone or derivatives thereof (and other polymerization inhibitor if necessary and hereinafter the same) may be dissolved so as to be a predetermined concentration. Phenothiazine or derivatives thereof and/or hydroquinone or derivatives thereof may be mixed in a pipe. When the concentration of formaldehyde in the (meth)acrylic acid solution extracted exceed the aforementioned predetermined range, it may be diluted with water, other solvent or the (meth)acrylic acid solution that is prepared at another step and has a little content of formaldehyde. Since a precipitation reaction of phenothiazine or derivatives thereof with formaldehyde proceeds easily at room temperature, each step such as dissolution (preparation of the polymerization inhibitor solution), preservation and transfer is preferably carried out at 15°C or more and 50°C or less (more preferably 30°C or less). On the other hand, since a precipitation reaction of hydroquinone or derivatives thereof with formaldehyde proceeds more slowly than in the case of phenothiazine or derivatives thereof, it is recommended that each step such as dissolution (preparation of the polymerization inhibitor solution), preservation and transfer is carried out at 15°C or more and 80°C or less (preferably 70°C or less, and more preferably 50°C or less).

The polymerization inhibitor solution prepared by the above-mentioned process can be utilized for inhibiting the polymerization of (meth)acrylic acid in all steps of the aforementioned steps (1) to (3) of the process for producing (meth)acrylic acid. It may be used for inhibiting the polymerization at a plural number of the steps. Furthermore, it can be used in a plural number of (meth)acrylic acid producing plants.

A form of the polymerization inhibitor solution when adding is not specifically limited. For example, the polymerization inhibitor solution with the predetermined concentration may be used on its own, or the polymerization solution may be diluted by the feed solution, the reflux solution and the like. In another word, it may be adequate to add necessary amount of the polymerization inhibitor solution for exhibiting the effect of the polymerization inhibition at each step.

In particular, it is preferable to feed the polymerization inhibitor solution to a column apparatus because temperature is high (around 50°C to 200°C) and it will become a condition of occurring polymerization of (meth)acrylic acid easily in the column apparatus. The column apparatus includes a collection column, a condenser, a distillation column and the like. The distillation column includes all distillation columns generally used for producing high purity (meth)acrylic acid such as a high boiling point component separation column for separating high boiling point components, a low boiling point component separation column for separating low boiling point components such as acetic acid and the like, an aldehyde removal column for removing unreacted acrolein and other aldehydes, and a maleic acid separation column for separating a by-product of maleic acid.

In the case that the polymerization inhibitor solution is used, for example, in the distillation column, it is desirable that the addition amount of phenothiazine is adjusted so as to be 10 ppm to 1000 ppm based on the amount of an evaporated (meth)acrylic acid when only phenothiazine is added to the polymerization inhibitor solution as the polymerization inhibitor.

It is also effective to use molecular oxygen in combination for the polymerization inhibition. As a feeding method of molecular oxygen, it is general that molecular oxygen is directly mixed with (meth)acrylic acid by bubbling and the like or indirectly mixed with (meth)acrylic acid by being dissolved in a solvent. For example, when distillation is adopted in the above-mentioned purification step (3), molecular oxygen can be blown as a gas state into a distillation column and a stripper from a bottom of the column and/or a reboiler. Molecular oxygen is preferably fed at a portion of 0.1 volumn % or more, since remarkable effect can not be obtained at less than 0.1 volume % of molecular oxygen. The feed amount of molecular oxygen is determined by a treatment capacity of a distillation apparatus, but when the feed amount is large, reconstruction of the distillation apparatus is usually required; therefore, it is preferable to feed it at a proportion of 1 volume % or less.

The present invention is the process for producing (meth)acrylic acid while preventing the polymerization of (meth)acrylic acid using the polymerization inhibitor solution which is prepared by the above-mentioned method in the aforementioned (meth)acrylic acid producing processes of the steps (1) to (3). In the present invention, the composition other than the essential composition of the present invention, i.e. the preparation and the utilization process of the polymerization inhibitor solution, can be adopted known processes as the process for producing (meth)acrylic acid including various modification and the like thereof.

### EXAMPLES

The present invention will be specifically explained below in detail by reference to Examples. However, the present invention is not limited to the following Examples, and modifications which do not depart from the spirit and scope of the present invention are all included in the present invention. Evaluation methods used in experiments below are as follows.

### Precipitation time

### (1) In the case of Experiment 1

Formaldehyde (HCHO: manufactured by Wako Pure Chemical Industry Ltd.: aqueous solution of 37 mass %) was added to 500 g of acrylic acid (manufactured by Wako Pure Chemical Industry Ltd.) from which a polymerization inhibitor originally added was removed by a simple distillation. The addition amount of HCHO is shown in Table 1. Ion exchange water was further added thereto so that the concentration of water was 10 mass % when the sum of acrylic acid, HCHO and water was referred to as 100 mass %. In cases of Experiment No.1-9 and No.1-10 that were reference examples, HCHO was not added, and thus water was added so that the concentration of water was 10 mass % when the sum of acrylic acid and water was referred to as 100 mass %. Phenothiazine (hereinafter, abbreviated as PTZ: manufactured by Wako Pure Chemical Industry Ltd.) was added to the solution which was stirred in a constant-temperature bath at 20°C and dissolved to obtain a polymerization inhibitor solution. The addition amount of phenothiazine is shown in Table 1. The polymerization inhibitor solution was kept at 20°C, and the time t1 at which a complete dissolution of PTZ was visually confirmed and the time t2 at which a precipitate was visually confirmed were measured. The result is shown as the precipitation time (the time from t1 to t2) in Table 1. The precipitation time was also measured for Experiment 3 and Experiment 4 by the same method described above and the result is shown in Table 3 and Table 4.

### (2) In the case of Experiment 2

5 mass % of furfural and 2 mass % of benzaldehyde were added to 465 g of acrylic acid which was removed a polymerization inhibitor, HCHO was added thereto, PTZ was added thereto while stirring in a constant-temperature bath at 35°C, and dissolved to prepare a polymerization inhibitor solution. The addition amount of HCHO and PTZ is shown in Table 2. Precipitation time was measured by the same method in Experiment 1 while keeping it at 35°C. The result is shown in Table 2. Experiment 2 is for confirming the influence of furfural and benzaldehyde that are by-products in the producing of acrylic acid.

### Amount of precipitate

Solutions that the condition of the concentration and the amount was the same as the above-described were left for 3 days in a constant-temperature bath. After leaving, each solution was filtered with a filter paper (manufactured by Advantec Co., Ltd.: No.2 filter paper; 150 mm in diameter) to obtain a filtrate and a precipitate on the filter paper. When the precipitate was observed, the precipitate was rinsed with ion exchange water and dried in a drying oven at 60°C for 24 hours. The weight of the dried precipitate was measured. The result is shown in Table 1 to Table 4

### Polymerization initiation time

About 10 mL of the filtrate obtained above was further filtered with a membrane filter (0.45 µm in pore size) to obtain a filtrate. 1 g of the filtrate was diluted with acrylic acid so as to be 10 ppm (based on mass) of PTZ. The amount of acrylic acid to dilute the filtrate is shown in Table 1 and Table 2. 10 ppm of PTZ means nearly the lower limit of a general PTZ concentration when PTZ is used in the process for producing (meth)acrylic acid. 5 mL of the solution was put in a test tube and deaerated for 3 minutes under the reduced pressure of 19998.3 Pa (150 mmHg). Then, the test tube was sealed and immersed in an oil bath at 95°C to measure the time (polymerization initiation time) that the solution became cloud from the test tube was immersed. Clouding of the solution was visually confirmed. The result is shown in Table 1 and Table 2.

In Experiment 3 and Experiment 4, 0.1 g of the filtrate which was filtered with a membrane filter (0.45 µm in pore size) of the above-obtained filtrate was diluted with acrylic acid. The amount of acrylic acid to dilute the filtrate is shown in Table 3 and Table 4. In Experiment 3 and Experiment 4, the concentration of hydroquinone or derivatives thereof (hydroquinone (HQ) or methoquinone (MQ): either of them was manufactured by Wako Pure Chemical Industry Ltd.) was adjusted so as to be 50 ppm (based on mass). The result is shown in Table 3 and Table 4.

### Experiment 1

The addition amount of HCHO and PTZ and the amount of acrylic acid (AA) for dilution at measuring polymerization initiation time were varied as shown in Table 1. The precipitation time, the amount of precipitate and the polymerization initiation time were measured by the method described above. The result is shown in Table 1.

### Experiment 2

The addition amounts of HCHO and PTZ and the amount of acrylic acid (AA) for dilution at measuring polymerization initiation time were varied as shown in Table 2. The precipitation time, the amount of precipitate and the polymerization initiation time were measured by the method described above. The result is shown in Table 2.

**Table 1**

| Experiment 1 | | | | | | |
|---|---|---|---|---|---|---|
| Experiment No | Addition amount of HCHO (ppm by mass) | Addition amount of PTZ (mass%) | Amount of AA for dilution (g) | Precipitation time (at 20°C) | Amount of precipitate (g) | Polymerization initiation time (at 95°C) |
| 1-1 | 10 | 1 | 1000 | No precipitation for 3 days | 0 | 19.5 Hours |
| 1-2 | 100 | 1 | 1000 | No precipitation for 3 days | 0 | 20 Hours |
| 1-3 | 500 | 1 | 1000 | 50 Hours | 0.05 | 18 Hours |
| 1-4 | 2000 | 1 | 1000 | 37 Hours | 0.36 | 15 Hours |
| 1-5 | 3500 | 1 | 1000 | 30 Hours | 0.45 | 12 Hours |
| 1-6 | 5700 | 1 | 1000 | 4.5 Hours | 1.79 | 4.5 Hours |
| 1-7 | 10000 | 1 | 1000 | 1 Hour | 5.48 | 20 Minutes |
| 1-8 | 10000 | 0.01 | 10 | No precipitation for 3 days | 0 | 18.5 Hours |
| 1-9 | 0 | 0 | 1000 | - | - | 18 Minutes |
| 1-10 | 0 | 1 | 1000 | No precipitation for 3 days | 0 | 20 Hours |

**Table 2**

| Experiment 2 | | | | | | |
|---|---|---|---|---|---|---|
| Experiment No. | Addition amount of HCHO (ppm by mass) | Addition amount of PTZ (mass%) | Amount of AA for dilution (g) | Precipitation time (at 35°C) | Amount of precipitate (g) | Polymerization initiation time (at 95°C) |
| 2-1 | 3500 | 2 | 2000 | No precipitation for 3 days | 0 | 22 Hours |
| 2-2 | 3500 | 3 | 3000 | 45 Hours | 0.67 | 17 Hours |
| 2-3 | 3500 | 5 | 5000 | 5.5 Hours | 10.62 | 6 Hours |

From the results of Experiments No. 1-1 to No. 1-5 (corresponding to examples) and Experiments No.1-6 to No.1-7 (corresponding to reference examples), it was cleared that the precipitation time was shortened and the precipitation amount was increased in accordance with the increase of the concentration of HCHO when the concentration of PTZ was constant. It was also confirmed that the polymerization initiation time was shortened in accordance with the increase of the concentration of HCHO and thus the effect of polymerization inhibition was lessened.

Furthermore, from the results of Experiment No.2-1 and No.2-2 (corresponding to examples) and Experiment No. 2-3 (corresponding to reference examples), it was cleared that the amount of precipitate was not so large and the effect of polymerization inhibition was hardly lowered even though 3 mass % of PTZ is added when the concentration of HCHO was within the predetermined range.

### Experiment 3

Formaldehyde was added to 500 g of acrylic acid which was removed a polymerization inhibitor by the same method described in Experiment 1, and ion exchange water was further added thereto so that the concentration of water was 80 mass % when the sum of acrylic acid, HCHO and water was referred to as 100 mass %. HQ was added to the solution which was stirred in a constant-temperature bath at the temperature (retention temperature) shown in Table 3, and dissolved. The addition amount of HCHO and HQ is shown in Table 3. The solution was kept at the temperature shown in Table 3, and the time t1 at which a complete dissolution of HQ was visually confirmed and the time t2 at which a precipitate was visually confirmed were measured. The result is shown as the precipitation time (the time from t1 to t2) in Table 3. Furthermore, the amount of precipitate and the polymerization initiation time were measured by the same method in Experiment 1 and the result is shown in Table 3.

**Table 3**

| Experiment 3 | | | | | | | |
|---|---|---|---|---|---|---|---|
| Experiment No. | Addition amount of HCHO (ppm by mass) | Addition amount of HQ (mass%) | Amount of AA for dilution (g) | Retention temperature (°C) | Precipitation time | Amount of precipitate (g) | Polymerization initiation time (hr) (at 95°C) |
| 3-1 | 10 | 8 | 1600 | 20 | No precipitation for 3 days | 0 | 24.0 |
| 3-2 | 100 | 8 | 1600 | 20 | No precipitation for 3 days | 0 | 24.5 |
| 3-3 | 500 | 8 | 1600 | 20 | No precipitation for 3 days | 0 | 24.5 |
| 3-4 | 2000 | 8 | 1600 | 20 | No precipitation for 3 days | 0 | 24.0 |
| 3-5 | 5000 | 8 | 1600 | 20 | No precipitation for 3 days | 0 | 22.0 |
| 3-6 | 10 | 8 | 1600 | 40 | No precipitation for 3 days | 0 | 24.5 |
| 3-7 | 100 | 8 | 1600 | 40 | No precipitation for 3 days | 0 | 24.5 |
| 3-8 | 500 | 8 | 1600 | 40 | 72 Hours | 0.03 | 23.5 |
| 3-9 | 2000 | 8 | 1600 | 40 | 69 Hours | 0.07 | 22.0 |
| 3-10 | 5000 | 8 | 1600 | 40 | 62 Hours | 0.58 | 18.5 |
| 3-11 | 10 | 8 | 1600 | 60 | 72 Hours | 0.08 | 23.0 |
| 3-12 | 100 | 8 | 1600 | 60 | 71 Hours | 0.27 | 22.0 |
| 3-13 | 500 | 8 | 1600 | 60 | 68Hours | 0.48 | 21.5 |
| 3-14 | 2000 | 8 | 1600 | 60 | 64 Hours | 0.81 | 19.0 |
| 3-15 | 5000 | 8 | 1600 | 60 | 39 Hours | 2.00 | 14.5 |
| 3-16 | 10 | 8 | 1600 | 80 | 70 Hours | 1.37 | 17.0 |
| 3-17 | 100 | 8 | 1600 | 80 | 69 Hours | 1.67 | 17.5 |
| 3-18 | 500 | 8 | 1600 | 80 | 46 Hours | 2.28 | 15.5 |
| 3-19 | 2000 | 8 | 1600 | 80 | 32 Hours | 3.12 | 12.0 |
| 3-20 | 5000 | 8 | 1600 | 80 | 7 Hours | 4.52 | 5.5 |
| 3-21 | 2000 | 10 | 2000 | 40 | 67 Hours | 0.37 | 21.5 |
| 3-22 | 2000 | 12 | 2400 | 40 | 63 Hours | 1.20 | 17.5 |
| 3-23 | 2000 | 10 | 2000 | 60 | 60 Hours | 1.29 | 18.5 |
| 3-24 | 2000 | 12 | 2400 | 60 | 41 Hours | 3.72 | 11.0 |
| 3-25 | 2000 | 12 | 2400 | 80 | 5 Hours | 6.47 | 3.5 |
| 3-26 | 5000 | 0.05 | 10 | 80 | No precipitation for 3 days | 0 | 21.5 |
| 3-27 | 5000 | 0.05 | 10 | 40 | No precipitation for 3 days | 0 | 23.0 |
| 3-28 | 0 | 10 | 2000 | 40 | No precipitation for 3 days | 0 | 25.5 |
| 3-29 | 0 | 10 | 2000 | 60 | No precipitation for 3 days | 0 | 25.0 |
| 3-30 | 0 | 10 | 2000 | 80 | No precipitation for 3 days | 0 | 25.0 |

### Experiment 4 (In the case of using both PTZ and hydroquinone or derivatives thereof)

Formaldehyde was added to 500 g of acrylic acid which was removed a polymerization inhibitor by the same method described in Experiment 1, and ion exchange water was further added thereto so that the concentration of water was 10 mass % when the sum of acrylic acid, HCHO and water was referred to as 100 mass %. PTZ and HQ or MQ were added to the solution which was stirred in a constant-temperature bath at the temperature (retention temperature) shown in Table 4, and dissolved. The addition amount of HCHO, HQ and MQ is shown in Table 4. The solution was kept at the temperature shown in Table 4, and the time t1 at which a complete dissolution of HCHO and HQ or MQ was visually confirmed and the time t2 at which a precipitate was visually confirmed were measured. The result is shown as the precipitation time (the time from t1 to t2) in Table 4. Furthermore, the amount of precipitate and the polymerization initiation time were measured by the same method in Experiment 1 and the result is shown in Table 4.

**Table 4**

| Experiment 4 | | | | | | | | |
|---|---|---|---|---|---|---|---|---|
| Experiment No. | Addition amount of HCHO (ppm by mass) | Addition amount of PTZ (mass%) | Addition amount of hydroquinone or derivatives thereof (mass%) | Amount of AA for dilution (g) | Retention temperature (°C) | Precipitatio n time (at 35°C) | Amount of precipitate (g) | Polymerization initiation time (at 95°C) |
| 4.1 | 2000 | 1 | HQ: 10 | 2000 | 40 | 68 Hours | 0.09 | 23 Hours |
| 4-2 | 6500 | 1 | HQ: 10 | 2000 | 40 | 5 Hours | 2.33 | 13 Hours |
| 4-1 | 2000 | 1 | MQ: 10 | 2000 | 40 | 70 Hours | 0.05 | 25 Hours |
| 4-2 | 6500 | 1 | MQ: 10 | 2000 | 40 | 8 Hours | 2.05 | 14 Hours |

### Experiment 5 (Experiment in a small scale facilities)

An experiment was carried out using acrylic acid distillation facilities shown in Fig. 1. A distillation column T-1 was a column with an inner diameter of 105 mm equipped with a distillate extraction pipe and a reflux solution feed pipe at the top portion of the column, a raw material feed pipe at a central portion (at 10th stage) of the column, a bottom liquid discharge pipe and an oxygen blowing pipe at a bottom portion of the column. Sieve trays made of stainless steel were set with 20 stages and a stage interval of 147 mm in the distillation column T-1. V-1 was a tank for a raw material liquid which was fed to the distillation column, P-1 was a pump for feeding the raw material liquid, Ex-2 was a condenser, V-2 was a distillate receiving tank, P-2 was a pump for feeding a reflux solution, and P-5 was a pump for discharging the distillate. An acrylic acid solution for a polymerization inhibitor solvent was discharged by the pump P-5.

The acrylic acid solution discharged was transferred to polymerization inhibitor solution tanks V-4 or V-5, and phenothiazine (if necessary, also other polymerization inhibitor) was added hereto. In the experiment, the polymerization inhibitor solution in which 1 mass % of phenothiazine was dissolved in 30 kg of acrylic acid manufactured by Wako Pure Chemical Industry Ltd. was stocked in the V-4 and fed to a gas phase at the upper portion of the condenser E-2 by the pump P-4 for 2 days from the start up of distillation. Subsequently, the polymerization inhibitor solution in which 1 mass % of phenothiazine was dissolved in the acrylic acid solution obtained from the distillate was prepared in the tank V-5, and fed to the condenser Ex-2 by the pump P-4. The distillate extracted from the top portion of the distillation column T-1 was mixed with the polymerization inhibitor solution in the condenser Ex-2, and the mixture was returned to the distillation column T-1 as a reflux solution by the pump P-2. Strainers St-41 and St-42 equipped with a screen of 200 meshes were provided between P-4 and V-4 or V-5.

B-1 was a reboiler, St-31 and St-32 were strainers equipped with a screen of 60 meshes, P-3 was a pump for circulating the bottom liquid of the column and V-3 was a tank for bottom liquid of the column.

In a distillation experiment, raw material liquid in which 81 mass % of acrylic acid, 3.7 mass % of acetic acid, 0.5 mass % of furfural, 9 mass % of water, 3.4 mass % of acrylic acid dimer, 35 ppm by mass of formaldehyde and formic acid and other by-products as a residue were included was fed to the bottom portion of the distillation column T-1 at 11.93 kg/hr. The polymerization inhibitor solution was fed from the condenser Ex-2 to the distillation column T-1 so as to be 200 ppm by mass based on the amount of evaporated acrylic acid. Furthermore, molecular oxygen was blown into the column T-1 so as to be 0.3 volume % based on the amount of evaporated acrylic acid. The amount of evaporated acrylic acid means the total quantity of acrylic acid evaporated from the bottom of the column, and the amount of acrylic acid evaporation corresponds to a heat quantity applied from the reboiler B-1.

Filtered residue (precipitate of polymer and/or phenothiazine or derivatives thereof) on screens of Strainers St-31, St-32, St-41 and St-42 was removed once a day, rinsed and weighted.

In a steady state operation (24 hours after the start of distillation), 70°C of a temperature at the top of the column T-1, 120°C of a temperature at the bottom of the column, 94 hPa of a pressure at the top the column and 0.5 of a reflux ratio (a total molar number of the reflux solution per unit time / a total molar number of the distillate per unit time) were set. The amount of distillate from the top of the column was 11.29 kg/hr, and the feed amount of the polymerization inhibitor solution was 0.34 kg/hr. A composition of the distillate was 85 mass % of acrylic acid, 4.0 mass % of acetic acid, 0.29 mass % of furfural, 9.8 mass % of water, 0.38 mass % of acrylic acid dimer, 38 ppm by mass of formaldehyde, and by-products such as formic acid as a residue.

24 hours after confirming the steady operation state (2 days after the start of distillation), the polymerization inhibitor solution in which phenothiazine was added to 100 kg of the distillate extracted from the distillation column T-1 so as to be 1 mass % concentration was prepared in the tank V-5, and a valve was switched so that the polymerization inhibitor solution from the tank V-5 was fed to the condenser Ex-2. The polymerization inhibitor solution was fed to the distillation column together with the reflux solution so as to be 200 ppm by mass based on the amount of evaporated acrylic acid. Then, when the polymerization inhibitor solution in the tank V-5 was lessened, the polymerization inhibitor solution was prepared using the distillate in the tank V-4 and fed to the distillation column T-1, and this operation was repeated to continue the distillation. 10 days after the polymerization inhibitor solution was changed to be prepared using the distillate, a slight amount of precipitate was confirmed in the tank V-5, and the distillation was continued.

After the operation for 30 days, the distillation was stopped. When the inside of the distillation column T-1 was investigated, the adherence of a polymer was not confirmed and the distillation column was in a state that the operation can be continued thereafter. The integration amount of the filtrated residue on the screens of the strainers St-31 and St-32 was 150 g for 30 days. And the integration amount of the filtrated residue on the strainers St-41 and St-42 was about 3 g for 30 days. The filtrated residues (polymer and precipitate) were weighted in a wet state.

### Experiment 6 (Experiment in a small scale facilities)

A distillation experiment was carried out in the same way as Experiment 5 except that a raw material liquid containing 1500 ppm by mass of formaldehyde (other composition was the same as Experiment 5) was used. 3 days after the polymerization inhibitor solution was changed to be prepared using the distillate, a slight amount of precipitate was confirmed in the tank V-5, and the distillation was continued. The concentration of formaldehyde in the distillate was 1650 ppm by mass.

After the operation for 30 days, the distillation was stopped. When the inside of the distillation column T-1 was investigated, the adherence of polymer was not confirmed and the distillation column was in a state that the operation can be continued thereafter. The integration amount of the filtrated residue on the screens of the strainers St-31 and St-32 was 230 g for 30 days. And the integration amount of the filtrated residue on the strainers St-41 and St-42 was about 50 g for 30 days. The filtrated residues (polymer and precipitate) were weighed in a wet state.

### Experiment 7 (Experiment for comparison)

A distillation experiment was carried out in the same way as Experiment 5 except that a raw material liquid containing 5000 ppm by mass of formaldehyde (other composition was the same as Experiment 5) was used. 1 day after the polymerization inhibitor solution was changed to be prepared using the distillate, a precipitate was confirmed in the tank V-5, and the distillation was continued. The concentration of formaldehyde in the distillate was 5530 ppm by mass.

5 days after the polymerization inhibitor solution was changed to be prepared using the distillate (7 days after the start of distillation), distillation was stopped because the fluctuation of the pressure at the top portion of the distillation column T-1 was enlarged. When the inside of the distillation column T-1 was investigated, the adherence of polymer was confirmed. The integration amount of the filtrated residue on the screens of the strainers St-31 and St-32 was 600 g for 7 days. And the integration amount of the filtrated residue on the strainers St-41 and St-42 was about 3.53 kg for 7 days. The filtrated residues (polymer and precipitate) were weighed in a wet state.

According to the present invention, even if a solution containing phenothiazine or derivatives thereof and/or hydroquinone or derivatives thereof is prepared using the (meth)acrylic acid solution inevitably containing formaldehyde which is produced in the process for producing (meth)acrylic acid, the precipitation as the reaction products of phenothiazine or derivatives thereof and/or hydroquinone or derivatives thereof with formaldehyde can be suppressed, and the stable polymerization inhibitor solution can be provided; therefore, inhibition of the (meth)acrylic acid polymerization can be achieved at any step in the process for producing (meth)acrylic acid, and (meth)acrylic acid can be produced stably. The polymerization inhibitor solution prepared by the present invention may be applied for preventing polymerization in the process for producing other vinyl compounds having a process in which formaldehyde is generated as a by-product.

## Claims

1. A process for producing (meth)acrylic acid while preventing a polymerization of (meth)acrylic acid using a polymerization inhibitor solution, comprising the steps of:
obtaining a (meth)acrylic solution containing formaldehyde from any step of the process for producing (meth)acrylic acid to use as a solvent of the polymerization inhibitor solution, and
adding phenothiazine or derivatives thereof and/or hydroquinone or derivatives thereof to the (meth)acrylic solution containing formaldehyde to obtain the polymerization inhibitor solution so that the polymerization inhibitor solution contains 0.1 mass % to 3 mass % of phenothiazine or derivatives thereof and/or 0.1 mass % to 10 mass % of hydroquinone or derivatives thereof,
wherein the polymerization inhibitor solution contains 10 ppm to 5000 ppm (based on mass) of formaldehyde.

2. The process for producing (meth)acrylic acid according to claim 1, wherein the polymerization inhibitor solution contains 10 ppm to 2000 ppm (based on mass) of formaldehyde.

3. The process for producing (meth)acrylic acid according to claim 1 or 2, wherein the (meth)acrylic acid solution containing formaldehyde comprises 1 mass % to 99.9 mass % of (meth)acrylic acid.

4. The process for producing (meth)acrylic acid according to any one of claims 1 to 3, further comprising
adding a polymerization inhibitor other than phenothiazine or derivatives thereof and hydroquinone or derivatives thereof to the (meth)acrylic solution containing formaldehyde to obtain the polymerization inhibitor solution.

5. The process for producing (meth)acrylic acid according to any one of claims 1 to 4, wherein the polymerization inhibitor solution is fed to a column apparatus used in the process for producing (meth)acrylic acid.

6. The process for producing (meth)acrylic acid according to claim 5, wherein the column apparatus is one or more of a collection column, a condenser and a distillation column.
